(19)

Europäisches
Patentamt
European
Patent Office
Office européen
des brevets

(11) **EP 2 487 617 A1**

(12) **EUROPEAN PATENT APPLICATION**

(43) Date of publication:
**15.08.2012 Bulletin 2012/33**

(51) Int Cl.:
***G06F 19/18*** (2011.01)

(21) Application number: **11186897.2**

(22) Date of filing: **27.10.2011**

<table>
<tr><td>

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.10.2010 KR 20100105504**

(71) Applicant: **Samsung SDS Co. Ltd.**
**Seoul (KR)**

</td><td>

(72) Inventors:
• **Yun, Hong-Seok**
 **Daejeon (KR)**
• **Sun, Choong-Hyun**
 **Gyeonggi-do (KR)**
• **Park, In-Ho**
 **Gyeonggi-do (KR)**

(74) Representative: **Grünecker, Kinkeldey,**
**Stockmair & Schwanhäusser**
**Leopoldstrasse 4**
**80802 München (DE)**

</td></tr>
</table>

(54) **Apparatus and method for extracting biomarkers**

(57) Provided are an apparatus and method for extracting biomarkers with higher reliability by analyzing toxicity indicating how genetic variants appearing in sequences affect gene functions. The apparatus includes a pre-processor that analyzes sequences of samples of genes and extracts data of genetic variants mapped to the genes, a toxicity prediction unit that obtains toxicity scores obtained by quantifying genetic dysfunctions affected by the data of genetic variants, and a modularization unit that searches for a least one sub-module including a set of genes whose toxicity scores exceed a predetermined critical value from a genetic network.

FIG. 1

EP 2 487 617 A1

Description

CROSS-REFERENCE TO RELATED APPLICATION

This application claims priority from Korean Patent Application No. 10-2010-0105504 filed on October 27, 2010 in the Korean Intellectual Property Office, and all the benefits accruing therefrom under 35 U.S.C. 119, the contents of which in its entirety are herein incorporated by reference.

BACKGROUND

1. Field of the Invention
The present invention relates to bioinformatics technology, and more particularly to an apparatus and method for extracting biomarkers with higher reliability by analyzing toxicity indicating how genetic variants appearing in sequences affect gene functions.

2. Description of the Related Art
The completion of the human genome project resulted in deciphering human DNA sequences, by which various human gene functions have been elucidated. In particular, as a variety of genetic variants were revealed and findings are that the genetic variants may cause differences in diverse human traits and may cause specific diseases, the human genome analysis and research are further accelerating. However, it is still difficult to find which one among a large volume of genetic variants occurring to human genomes may actually cause diseases.

Recently, Next Generation Sequencing (NGS) as one alternative approach for overcoming the difficulty, have been researched. The NGS has enabled base sequencing of the entire genome of an individual. In addition, it has become possible to extract disease-specific genetic variants by comparative analysis of base sequences and variants in a disease group and a normal group.

Meanwhile, another research into Genome Wide Association Study (GWAS) analysis technology has also been made based on statistical analysis of populations using Single Nucleotide Polymorphism (SNP) chips, instead of base sequencing. According to the GWAS analysis technology, significant genetic variants frequently occurring in a specific disease group can be extracted by analyzing SNP data obtained from several thousands to thousands to tens of thousands of people. However, even if the genetic variants are extracted by a variety of analysis methods, additional experiments should be carried out in ascertaining whether genes including the genetic variants are actually expressed or genetic dysfunctions are caused, which may incur a considerable loss in time and cost. In addition, various causes of specific diseases cannot be fully explained by using only information on individual genetic variants.

To overcome the disadvantages, another technology is also researched to analyze interactions among components of biological systems and to decode the biological systems based on the analysis result, which is called systems biology. That is to say, a biological function manifested from a gene is merged with a function manifested from another gene to cooperatively act for performing vital functions of life while maintaining biological homeostasis even if incessant changes occur to the external environment. This technology entails analysis of functional location and interactions of genes having genetic variants based on network analysis of biological components, and provides a better understanding how genetic variants exert effects on surrounding components and how the effects propagate. In addition, this technology provides grounds for explaining connectivity between genetic variants and known gene interactions, gene regulation circuits, protein interactions, metabolism, signal transmission circuits.

Various intracellular processes required for normal cellular activities are actuated as a group of functional modules, which are smaller, more specific proteins or genes. A series of methods for predicting toxicity in protein functions, generated by individual Non-Synonymous Single Nucleotide Polymorphism (nsSNP) in individual genes have been proposed, including Sorting Tolerant From Intolerant (SIFT), Polymorphism Phenotyping (PolyPhen), Map Annotator and Pathway Profiler (MAPP), and so on. However, the proposed methods are problematic because they are limited in finding out causes of high-complexity diseases or disease markers.

Generall y, a proportion of causative SNPs that are toxic to protein functions is very low. Thus, in Gene Set Enrichment Analysis (GSEA) and SNP analysis, since all kinds of data estimated using SNPs are used irrespective of whether the SNPs influence toxicity to protein functions or not, there is a high probability of misjudging that biological pathways or a set of genes, which are not actually closely related with a specific disease, are considered as being statistically significant. Accordingly, it is necessary to develop techniques for accurately identifying biomarkers related to specific diseases by analyzing disease-specific genetic variants based on a biomolecular network and manifestation pattern analysis of genes belonging to the biomolecular network.

SUMMARY

To overcome the limitations with the conventional art in which interaction modularization and analysis are carried out using only a partial proportion of genetic variants or gene manifestation patterns, the present invention is provided to detect highly reliable biomarkers by analyzing toxicity indicating how genetic variants appearing in sequences affect gene functions.

The present invention is also provided to develop a toxicity prediction method of quantifying vitally influential toxicity in multiple manners in detecting the biomarkers.

These and other objects of the present invention will be described in or be apparent from the following description of the preferred embodiments.

According to an aspect of the present invention, there is provided an apparatus for extracting causal biomarkers of a specific disease by analyzing how genetic variants appearing in sequences affect gene functions, the apparatus including a pre-processor that analyzes sequences of samples of genes and extracts data of variants mapped to the genes, a toxicity prediction unit that obtains toxicity scores obtained by quantifying genetic dysfunctions affected by the data of variants, and a modularization unit that searches for at least one sub-module including a set of genes whose toxicity scores exceed a predetermined critical value from a genetic network.

According to another aspect of the present invention, there is provided an apparatus for predicting toxicity scores for quantifying genetic dysfunctions affected by data of variants appearing in sequences of genes, the apparatus including a toxicity calculation unit that applies the data of variants to a plurality of toxicity prediction models to obtain the respective toxicity scores, and assigns weights to the respective toxicity scores to obtain weighted toxicity scores, a significance calculation unit that calculates significance of a corresponding genetic variant based on the frequency of the data of variants, and a score computation unit that combines the weighted toxicity scores and the significance and computes toxicity scores.

According to a particular embodiment the toxicity calculation unit comprises:

a feature vector generation unit that generates feature vectors including various factors from the data of genetic variants;
an adapter that sorts factors necessary for the respective prediction models from the generated feature vectors;
two or more prediction models that receive the sorted factors to detect individual non-synonymous single nucleotide polymorphism (nsSNP) in protein sequences; and
a weight assignment unit that assigns weights to outputs of the prediction models and sums the weights.

According to a particular embodiment the weight assignment unit normalizes the outputs of the prediction models to values ranging between 0 and 1, multiplies the normalized outputs by weights, sums the multiplication results, and normalizes the summing result to a value ranging between 0 and 1.

According to a particular embodiment the feature vector includes at least two of conservation scores of amino acids at positions of genes and proteins mapped to genetic variants in various biological species, biochemical hydrophobicity resulting from amino acid substitution, a change in protein structural features, presence or absence of intron splice junction sites, and five prime untranslated region (5'-UTR) variation position.

According to a particular embodiment each of the prediction models includes at least one of Sorting Tolerant From Intolerant (SIFT), Polymorphism Phenotyping (PolyPhen), and Map Annotator and Pathway Profiler (MAPP)

According to a particular embodiment the significance calculation unit calculates the significance based on the probability of detecting a genetic variant of the corresponding gene from the disease group variants, and the probability is obtained by maximum likelihood estimation or Bayesian probability estimation.

According to a particular embodiment the score computation unit obtains a final toxicity score by dividing a sum of toxicity scores of the genetic variants in a single gene by a gene length.

According to still another aspect of the present invention, there is provided a method for extracting causal biomarkers of a specific disease by analyzing how genetic variants appearing in sequences of genes affect gene functions, the method including obtaining toxicity scores obtained by quantifying genetic dysfunctions based on data of variants included in the genes, searching for a plurality of sub-modules as a set of genes whose toxicity scores exceed a predetermined critical value from a genetic network, and determining an order of priority in the searched plurality of sub-modules.

According to a particular embodiment the determining of the order of priority comprises determining the order of priority by assigning a higher priority to a sub-module having a higher Z-score among Z-scores of the plurality of sub-modules.

According to a particular embodiment the method comprises merging proteins manifested from the genes from which the toxicity scores are obtained by the toxicity prediction unit with proteins from the known interaction database to generate an interaction network.

According to a particular embodiment the method further comprises evaluating functional relevance by comparing the sub-modules arranged by the order of priority with the known pathway database.

According to a further aspect of the present invention, there is provided a method for predicting toxicity scores for quantifying genetic dysfunctions affected by data of variants appearing in sequences of genes, the method including generating feature vectors including various factors from the data of variants, sorting factors necessary for the respective prediction models from the generated feature vectors, receiving the sorted factors to detect individual Non-synonymous Single Nucleotide Polymorphism (nsSNP) in protein sequences, and assigning weights to outputs of the prediction models and summing the weights to obtain weighted toxicity scores.

According to a particular embodiment the weights are empirically obtained using known disease genetic variants as learning data.

According to a particular embodiment the obtaining of the weighted toxicity scores comprises normalizing the outputs of the prediction models to values ranging between 0 and 1, multiplies the normalized outputs by weights, sums the multiplication results, and normalizes the summing result to a value ranging between 0 and 1.

According to a particular embodiment the method further comprises:

calculating significance of a corresponding genetic variant based on the frequency of the data of genetic variants; and combining the weighted toxicity scores and the significance and computing toxicity scores.

According to a particular embodiment the calculating of the significance comprises calculating the significance by a probability of detecting genetic variants of the corresponding gene from the disease group variants, the probability obtained based on maximum likelihood estimation or Bayesian probability estimation.

According to a particular embodiment the method comprises obtaining a final toxicity score by dividing a sum of toxicity scores of the genetic variants in a single gene by a gene length.

As described above, according to the embodiments of the present invention, it is possible to predict a genetic functional change or genetic dysfunctions, which may be caused by disease-specific sequence variants obtained by comparing variants in a disease group with variants in a normal group. In addition, biomarkers based on disease mechanism can be extracted by offering information on effects of individual genetic dysfunctions on interactions occurring in the entire biological system.

The biomarkers can be widely used in diagnosis of specific diseases, development of drugs for treatment of specific diseases and prevention of adverse effects.

## BRIEF DESCRIPTION OF THE DRAWINGS

The above and other features and advantages of the present invention will become more apparent by describing in detail preferred embodiments thereof with reference to the attached drawings in which:

FIG. 1 is a block diagram of an apparatus for extracting biomarkers according to an embodiment of the present invention;

FIG. 2 is a detailed block diagram of a pre-processor shown in FIG. 1;

FIG. 3 is a detailed block diagram of a toxicity prediction unit shown in FIG. 1;

FIG. 4 is a detailed block diagram of a toxicity calculation unit shown in FIG. 3;

FIG. 5 illustrates an exemplary mapping function used in the toxicity calculation unit shown in FIG. 4;

FIG. 6 is a flowchart illustrating a detailed process of searching for sub-modules by means of a modularization unit; and

FIG. 7 is a conceptual diagram for verifying significance from the number of genes commonly existing in a gene sub-module and a specific gene set.

## DETAILED DESCRIPTION OF THE EMBODIMENTS

The present invention will now be described more fully hereinafter with reference to the accompanying drawings, in which preferred embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art. The same reference numbers indicate the same components throughout the specification.

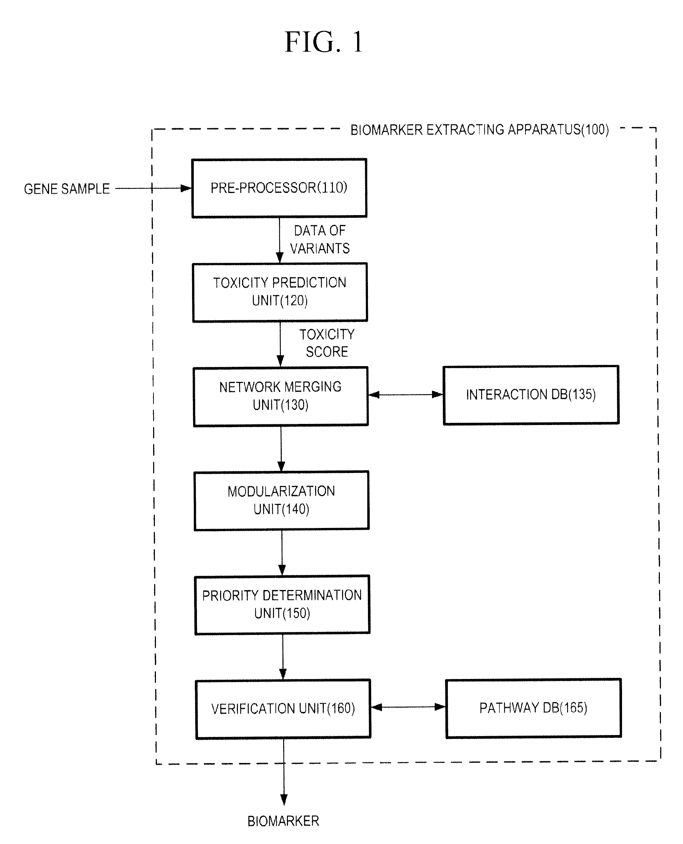

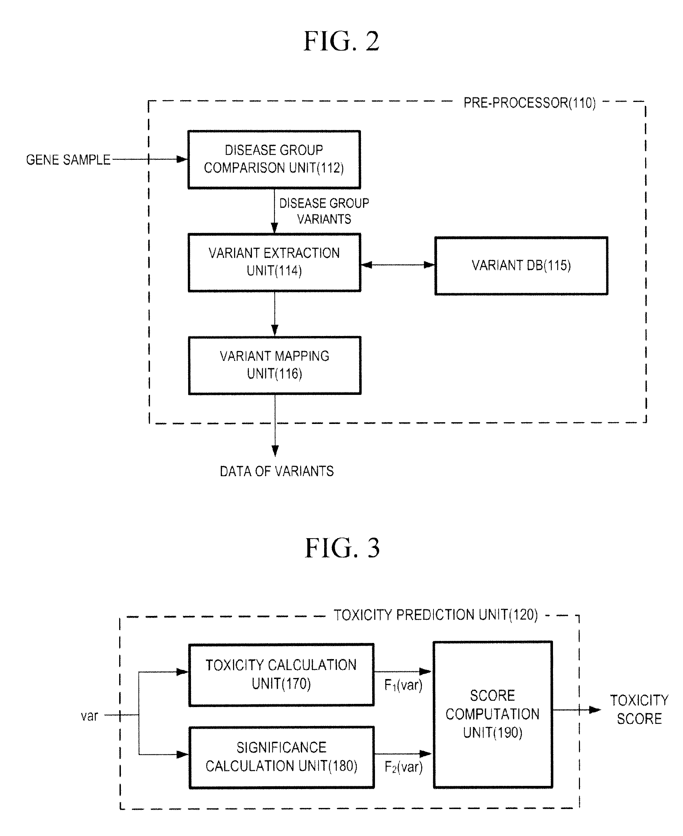

Hereinafter, an apparatus for extracting biomarkers according to an embodiment of the present invention will be described with reference to FIG. 1. FIG. 1 is a block diagram of an apparatus for extracting biomarkers according to an embodiment of the present invention. The biomarker extracting apparatus 100 may include a pre-processor 110, a toxicity prediction unit 120, a network merging unit 130, a modularization unit 140, a priority determination unit 150, and a verification unit 160. In some embodiments, the network merging unit 130 and the verification unit 160 may not be provided. In addition, interaction database 135 is linked with the network merging unit 130, and a pathway database 165 is linked with the verification unit 160. The functional blocks shown in FIG. 1 (also in FIGS. 2 to 4) may operate in a hardware system, which include a personal computer (either a portable type computer or a desk-top computer), or a server-client device connected to a communication network. For example, the functional blocks may be implemented in a software module to operate in a hardware system including a processor and a memory. The memory loads modules for the functional blocks to provide the loaded modules to the processor. The processor 110 processes the loaded modules to implement the biomarker extracting apparatus 100.

The pre-processor 110 extracts data of variants mapped to genes from gene samples. In detail, as shown in FIG. 2, the pre-processor 110 may include a disease group comparison unit 112, a variant extraction unit 114, a variant database 115 and a variant mapping unit 116.

In detail, the disease group comparison unit 112 extracts variants in a disease group and variants in a normal group, compares the disease group variants with the normal group variants, and acquires the disease group variants from gene samples. The variant extraction unit 114 extracts only new variants from the acquired disease group variants by referring to the variant database 115 that is known in the related art. In addition, the variant mapping unit 116 extracts only ones among the extracted new variants, the ones with amino acid changes made when they are expressed in proteins, that is, only non-synonymous variants, and maps the same to functional genes.

The data of variants of genes having genotypes analyzed from sequencing data is usually stored in GFF3 or GVF files. Currently, Genetic Feature Format version 3 (GFF3) is most widely used. Table 1 summarizes an example of data of variants indicated in GFF3 files.

Table 1

| Chr | Source | Type | Start | End | Score | Strand | Phase | Attributes |
|-----|--------|------|-------|-----|-------|--------|-------|------------|
| Chr1 | diBayes | SNP | 10492 | 10492 | 0.006 | . | . | genotype=Y;reference=C;... |
| Chr1 | diBayes | SNP | 28563 | 28563 | 0.000 | . | . | genotype=G;reference=A;... |
| Chr1 | diBayes | SNP | 11861 | 11861 | 0.000 | . | . | genotype=Y;reference=T;... |
| | | | 7 | 7 | | | | |

The variant data includes information regarding chromosome numbers (Chr) of genetic variants, variation start position (Start) and variation end position (End) of corresponding chromosomes, reference genotypes at corresponding chromosome position (reference), and attributes containing target genotypes and additional information. If the genotype information contains heterozygocity, two base sequences are at a time expressed using symbol Y.

Data of genetic variants specific to a corresponding disease can be obtained by removing data of variants in a normal group and known data of variants (for example, dbSNP, 1000 genome project, etc.) from data of variants for a specific disease group.

In such a manner, the disease group comparison unit 112 acquires variants existing in the disease group. The variant extraction unit 114 extracts only new variants from the acquired disease group variants by referring to data available from the known variant database 115.

The variant mapping unit 116 maps the new variants to genes known to the data of variants specific to the corresponding disease, and extracts information on whether each genetic variant is situated in an Intron region of a corresponding gene, whether there is an amino acid change in a protein expressed by the corresponding gene, or whether a STOP codon is generated.

Table 2 shows an example of data of variants mapped to genes.

Table 2

| Chr | Start | End | Gene ID | Variation region | Protein ID | Amino acid change | Heterozygocity |
|-----|-------|-----|---------|------------------|------------|-------------------|----------------|
| Chr1 | 1640667 | 1640667 | 984 | Coding | NP_277028 | K105K | Heterozygote |
| Chr1 | 3227034 | 3227034 | 63976 | Intron | . | . | Homozygote |
| Chr1 | 246195643 | 246195643 | 391191 | Coding | NP_ 001004491 | V203M | Homozygote |
| Chr1 | 246856127 | 246856127 | 127077 | Coding | NP_ 001001964 | Q309R | Heterozygote |

In Table 2, for example, K105K, means a variation without an amino acid change in protein even with a base sequence change, and V203M means that V is substituted by M in a 203th protein sequence. In addition, since an intron region is a non-coding region, there is no information regarding a protein ID and an amino acid change. "NP_xxxxxx", which is a type of a protein sequence ID, is a reference sequence ID (refseq ID) of The National Center for Biotechnology Information (NCBI) of U.S.A.

Referring again to FIG. 1, the data of variants extracted by the pre-processor 110 is offered to the toxicity prediction unit 120. The toxicity prediction unit 120 quantifies genetic dysfunctions of the corresponding gene based on the data of variants. The data of variants includes information on a genetic variant mapped to the gene, specifically, a variant causing amino acid substitution in a protein coding region.

As shown in FIG. 3, the toxicity prediction unit 120 includes a toxicity calculation unit 170, a significance calculation unit 180 and a score computation unit 190.

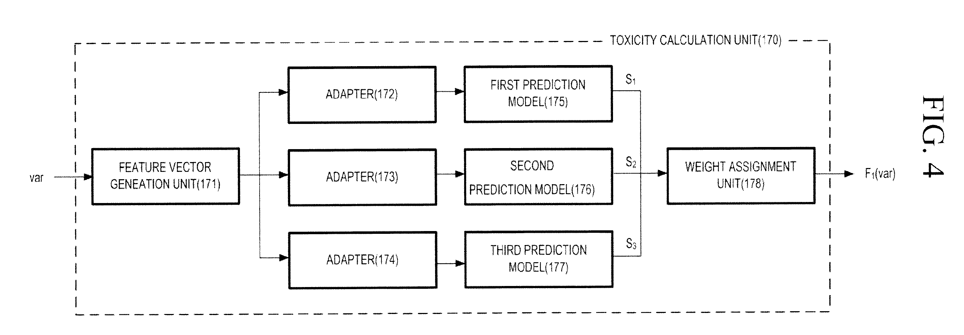

The toxicity calculation unit 170 applies input data of variants (var) to a plurality of toxicity prediction models to obtain respective toxicity scores, assigns weights to the respective toxicity scores, sums the assigned weights, and obtains a toxicity (weighted toxicity) of the data of variants. FIG. 4 is a detailed block diagram of a toxicity calculation unit shown in FIG. 3.

A feature vector generation unit 171 generates feature vectors including various components from the input data of variants. The components of the feature vectors include conservation scores of amino acids at positions of genes and proteins mapped to genetic variants in various biological species, biochemical hydrophobicity resulting from amino acid substitution, a change in protein structural features (protein interaction interface change, amino acid size, etc.), presence or absence of intron splice junction sites, and five prime untranslated region (5'-UTR) variation position.

The adapters 172, 173 and 174 sort factors necessary for the respective prediction models 175, 176 and 177 from the generated feature vectors, and offer the sorted factors to the corresponding prediction models 175, 176 and 177. The prediction models are obtained by conventional techniques researched for finding individual Non-synonymous Single Nucleotide Polymorphisms (nsSNP) in protein sequences. The nsSNP means a gene variant causing substitution of amino acids. Since the nsSNP may adversely affect intrinsic protein functions, it is taken into serious consideration. Examples of the prediction model may include Sorting Tolerant From Intolerant (SIFT), Polymorphism Phenotyping (PolyPhen), Map Annotator and Pathway Profiler (MAPP), and others. While FIG.4 illustrates 3 prediction models, an arbitrary number of prediction models may also be used.

Hereinafter, representative prediction models, SIFT, PolyPhen and MAPP, among currently known prediction models, will be briefly described.

The SIFT prediction model presumes that important amino acids will be conserved in the protein family, and so changes at well-conserved positions tend to be predicted as deleterious. In the SIFT prediction model, input sequences and similar protein sequences are acquired from protein sequence database, and Position Specific Scoring Matrices (PSSMs) are generated using the acquired sequences. Conservation scores of respective amino acid sequences of the input sequences, hydrophobicity of amino acids, and a probability of amino acids at sequence positions, are calculated to obtain toxicity to amino acid substitutions.

However, according to the SIFT prediction model, when input protein sequences are very similar to other sequence proteins collected by sequence similarity search, the generated PSSMs tend to appear well-conserved, which may lead to a high false prediction error where functionally non-deleterious amino acid substitutions are predicted to be highly tolerated. The SIFT prediction model demonstrates approximately 69% in sensitivity and approximately 13% in specificity.

A more sophisticated prediction model, called PolyPhen, has become available to predict toxicity by amino acid substitutions by combining sequence similarity, protein feature data and protein structure data. The PolyPhen prediction model uses Swiss-Prot annotations with the feature table and protein structure in addition to sequence conservation data used by SIFT. The PolyPhen prediction model predicts toxicity of amino acid substitutions by combining PSIC score difference values, amino acid substitution sites and substitution types. The PolyPhen prediction model demonstrates approximately 68% in sensitivity and approximately 16% in specificity.

A Map Annotator and Pathway Profiler (MAPP) has been developed to predict amino acid substitution similarity by combining protein sequence similarity and physiochemical features of amino acids. The MAPP performs sequence alignment using protein families showing sequence similarity and predicts all possible amino acid substitutions that may affect protein functions in consideration of a sequence difference of amino acids at the respective positions and physiochemical features (hydrophobicity, polarity, volume, etc) of amino acids.

As described above, individual nsSNPs are searched from protein sequences using the prediction models 175, 176 and 177, thereby obtaining scores of corresponding genetic variants. The scores $s_1$, $s_2$ and $s_3$ obtained by the respective prediction models are supplied to a weight assignment unit 178.

The weight assignment unit 178 normalizes the respective scores $s_1$, $s_2$ and $s_3$ to values between 0 and 1, multiplies the normalized scores by weights, and sums the multiplication results to obtain toxicity $F_1(var)$. The weights are values that are empirically obtained using known disease genetic variants as learning data. Therefore, the toxicity $F_1(var)$ can be computed by equation (1):

$$F_1(var) = \sum s_i \times w_i \qquad \dots(1)$$

The weight assignment unit 178 may further normalize the computed toxicity to a value between 0 and 1.

Meanwhile, since genetic variants occurring at repeated positions in multiple samples included in the disease group are determined as important variants, significance can be determined according to the frequency of the respective genetic variants.

Referring back to FIG. 3, the significance calculation unit 180 calculates significance of a corresponding genetic variant based on the frequency of the genetic variant, that is, probability distribution. The probability p(var) of the genetic variant means a probability that the corresponding genetic variant is found in the disease group samples, and may be obtained by, for example, by maximum likelihood estimation or Bayesian probability estimation.

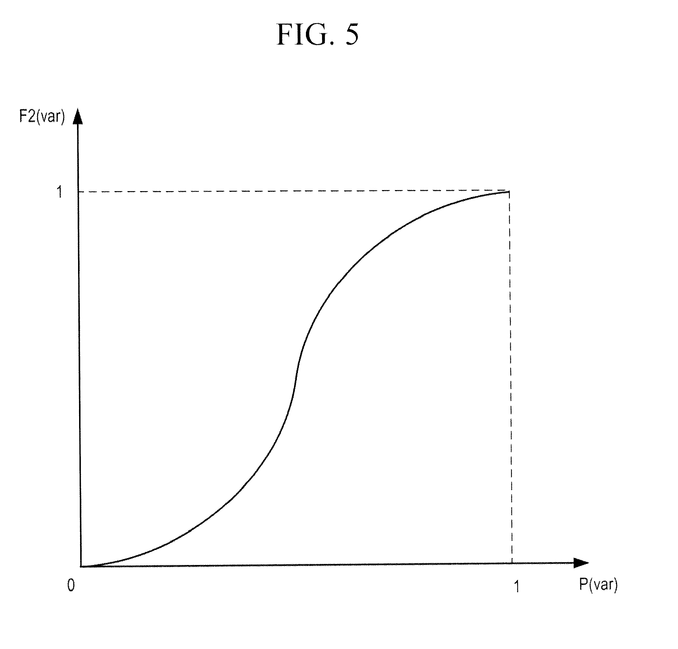

The thus obtained probability p(var) can be directly used as the significance. However, in order to use the probability p(var) as the significance in practice, it should be modified using a mapping function. The mapping function is a function for converting the probability p(var) between 0 and 1 to significance $F_2(var)$ between 0 and 1, as shown in FIG. 5. The mapping function may be set in various types. Preferably, as shown in FIG. 5, the mapping function has a relatively small slope around 0 and 1 and a relatively large slope around 0.5. That is to say, the significance has higher sensitivity around 0.5 than the probability around 0 and 1. For example, the mapping function may be defined by Equation (2):

$$F_2(Var) = \frac{1}{1 + e^{-\alpha \times p(Var)}} \qquad \dots (2)$$

where $\alpha$ is a constant.

The toxicity (a value between 0 and 1) obtained by the toxicity calculation unit 170 and the significance (a value between 0 and 1) obtained by the significance calculation unit 180 are finally supplied to the score computation unit 190. The score computation unit 190 combines the toxicity and the significance and computes a final toxicity score. For example, the toxicity score f(var) can be obtained by summing the toxicity and the significance, as defined by Equation (3), but not limited thereto:

$$f(Var) = F_1(Var) + F_2(Var) \qquad (3)$$

That is to say, the toxicity score f(var) can be obtained using various equations reflecting at least one of the toxicity and the significance. That is to say, the toxicity and the significance may lead to desirable effects when they are used together. Alternatively, the toxicity and the significance may also be independently used.

As described above, each genetic variant is mapped to a specific gene to be used to predict the toxicity of each gene. Here, although a single genetic variant that exerts a major effect is significant, a gene containing multiple genetic variants that exert relatively small effects are also considered as being significant. Thus, the score computation unit 190 may compute a final toxicity score by dividing toxicity scores f(var) of genetic variants contained in a single gene by a gene length. In this case, the final toxicity score s(Gene) can be obtained as defined by Equation (4):

$$s(Gene) = \frac{\sum_{Var \in Gene} f(Var)}{\text{gene length}} \qquad \dots(4)$$

A sum Σf(var) of toxicity scores of genetic variants existing in a single gene is divided by a gene length, thereby obtaining the final toxicity s(Gene). This suggests that not only a single genetic variant exerting a major effect but also multiple genetic variants are comprehensively considered. This also suggests that as the gene length is smaller, the final toxicity score may become larger when the toxicity score sum Σf(var) is a given value. That is to say, it can be presumed that a genetic variant with a higher toxicity score per unit gene length demonstrates a more significant toxicity with respect to a corresponding gene.

Referring back to FIG. 1, the network merging unit 130 merges proteins manifested from the genes whose toxicity scores are obtained by the toxicity prediction unit 120 with proteins known from the interaction database 135 to generate an interaction network.

In general, the actually expressed genetic variants may be protein units demonstrating biofunctions. That is to say, even if the genetic variants are deleterious, potential toxicity may not be expressed in actual protein units. Various manifestation types may be expressed by combination of various genetic variants. In the interaction network, combinations occur in the order of genes, proteins and enzymes, and the number of gene nodes may increase. A combination process of the interaction network is described in further detail in, for example, Automated Network Analysis Identifies Core Pathways in Clioblastoma (www.plosone.org, February 2010, volume 5, issue 2, e8918). In the present invention, if it is intended to obtain toxicity only in unit of genes, the combination process of the interaction network may be omitted.

The modularization unit 140 searches for a sub-module from a genetic network on which genes whose toxicity scores exceed a predetermined critical value are heavily populated. In more detail, the modularization unit 140 statistically evaluates a heavily populated distribution to search for a sub-module from a genetic network on which genes whose toxicity scores exceed a predetermined critical value are heavily populated. As an example of the statistical evaluation method, a hypergeometic distribution may be used.

Assuming that N represents a total number of genes on the genetic network, n represents the number of genes whose toxicity scores exceed a predetermined critical value, and m represents the number of genes existing in the sub-module of the genetic network, a probability P(X=k) of k, that is, the number of genes whose toxicity scores exceed a predetermined critical value in the sub-module of the genetic network can be computed by Equation (5):

$$P(X=k) = \frac{\binom{m}{k}\binom{N-m}{n-k}}{\binom{N}{n}} \quad \ldots(5)$$

where $\binom{N}{n}$ represent s the number of n combinations among N genes, that is, $_N C_n$.

Therefore, the probability(p) of k, that is, the number of genes whose toxicity scores exceed a predetermined critical value in the sub-module of the genetic network can be computed by Equation (6):

$$p = 1 - \sum_{i=0}^{k} P(x=i) \quad \ldots(6)$$

The probability (p) value means a probability of the number of genes whose toxicity scores exceed a predetermined critical value in the number k of genes existing in a particular sub-module. The critical value may be determined in various manners. In an example, in a toxicity score distribution of the overall genes, the critical value may be determined based on a predetermined percentile (e.g., 1 percentile, 5 percentile, 10 percentile, etc.). As described above, the higher the probability (p) for a particular sub-module, the more significant the sub-module.

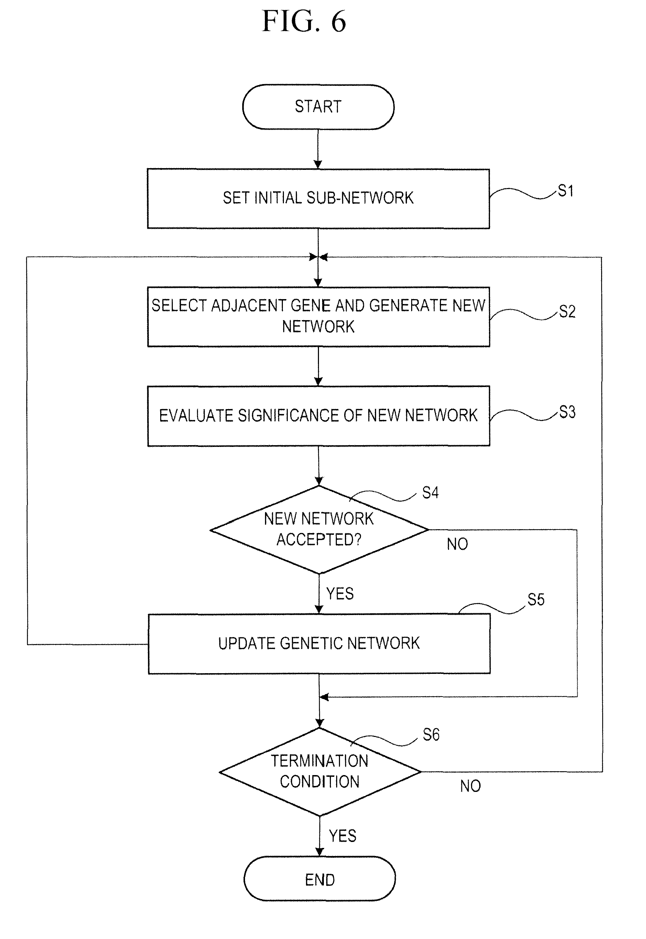

The modularization unit 140 may practically search for sub-modules using conventionally known greedy search algorithm or probabilistic search algorithm (e.g., simulated annealing), which will be described in detail with reference to FIG. 6.

First, the modularization unit 140 sets an initial sub-network (S1). The initial network means a network having all genes having significant toxicity scores (for example, genes having upper 5% of toxicity scores) as single nodes. The search algorithm is applied from nodes constituting the initial network to search for a sub-module of the genetic network having the optimum significance.

The modularization unit 140 selects an adjacent gene (a gene directly connected to a current gene) and merges the selected adjacent gene with the current gene to generate a new network (S2). Then, significance of the new network is evaluated (S3). That is to say, adjacent genes of the initial nodes are merged as new nodes to generate a new network, and significance of a unit composed of the merged nodes (a step of providing for a sub-module) is then evaluated. The significance may be evaluated by, for example, the probability (p) in the above-described hypergeometric distribution.

If the new network is significant (YES in step S4), the modularization unit 140 updates the current network to the significant network (S5), and the process proceeds to step S2. If the new network is not significant (NO in step S4), it is checked whether a termination condition is met without updating the network (S6), and if the termination condition is met (YES in step S6), the sub-module searching is terminated. If the termination condition is not met (NO in step S6), the process proceeds to step S2.

If the network updating process is terminated, sub-modules included in the finally updated genetic network may be determined (searching completed).

Referring again to FIG. 1, the priority determination unit 150 determines an order of priority in the plurality of sub-modules searched by the modularization unit 140. That is to say, the priority determination unit 150 determines the order of priority in the plurality of sub-modules by evaluating the correlation between changes in gene manifestation data and the respective sub-module of the genetic network found based on genetic variants.

Gene manifestation patterns of searched sub-modules are preferably analyzed on the corresponding sub-modules and genes directly connected thereto. This is because when a variation occurs to a gene such as a transfer regulatory factor, a change is more likely to occur to an manifestation pattern of a target gene of the transfer regulatory factor changed than to the transfer regulatory factor.

The gene manifestation data investigated in a normal group and in a disease group are pre-processed so that an manifestation difference between the normal and disease groups can be computed in Z-scores. For example, assuming that G is a set of genes directly connected to each sub-module, an index (es) for evaluating priority of a sub-module can be computed by Equation (7):

$$es = \frac{\sum_{i \in G} z_i}{\sqrt{|G|}} \quad \ldots (7)$$

where $z_i$ means a Z-score value of a toxicity score of each gene in the set of genes directly connected to a corresponding gene sub-module, and $|G|$ means a set size of genes (that is, the number of genes) directly connected to the corresponding gene sub-module. As known in the statistics field, the Z-score is a value obtained by subtracting a mean ($\mu$) from a current variable (x) and dividing the subtraction result by a standard deviation, and indicates how many standard deviations ($\sigma$) a current toxicity score value is above or below the mean ($\mu$). Eventually, the sub-modules arranged by the order of priority obtained by the above-described process may function as biomarkers indicating correlation to manifestation of a particular gene. Therefore, it is possible to predict how disease-specific sequence variants, obtained by comparison of the disease group and the normal group, change genetic functions of a corresponding gene or how genetic dysfunctions are caused. Further, information on effects of individual genetic dysfunctions on interactions in the entire biological system can be offered.

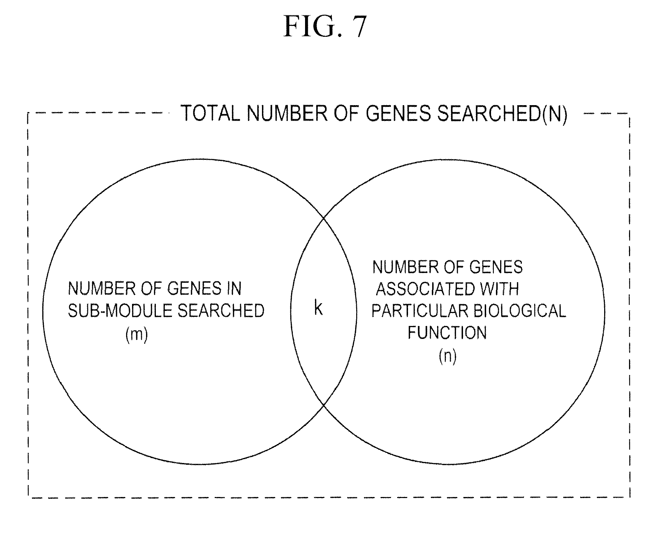

The verification unit 160 evaluates functional relevance of the sub-modules by comparing the sub-modules arranged by the order of priority with the known pathway database 165. Here, hypergeometric distribution is most widely used. In the evaluation method using hypergeometric distribution, significance of the number of genes repeatedly occurring to the set of genes extracted by biological function from pathway database 165 is computed for each sub-module. That is to say, as shown in FIG. 7, a significance probability is computed based on a total number N of genes searched, the number n of genes in a set of genes associated with a particular biological function, the number m of genes existing in the sub-module of the genetic network, and the number k of genes commonly existing in the gene sub-module and the set of genes associated with the particular biological function. The higher the probability, the more significant the final sub-module.

Each component described above with reference to FIGS. 1 through 4 may be implemented as a software component, such as a task, a class, a subroutine, a process, an object, an execution thread or a program performed in a predetermined region of a memory, or a hardware component, such as a Field Programmable Gate Array (FPGA) or Application Specific Integrated Circuit (ASIC). In addition, the components may be composed of a combination of the software and hardware components. The components may be reside on a computer-readable storage medium or may be distributed over a plurality of computers. Functions provided in the respective components may be separated into further detailed components or combined into one component performing a plurality of functions.

While the present invention has been particularly shown and described with reference to exemplary embodiments thereof, it will be understood by those of ordinary skill in the art that various changes in form and details may be made therein without departing from the spirit and scope of the present invention as defined by the following claims. It is therefore desired that the present embodiments be considered in all respects as illustrative and not restrictive, reference being made to the appended claims rather than the foregoing description to indicate the scope of the invention.

**Claims**

1. An apparatus (100) for extracting causal biomarkers of a specific disease by analyzing how genetic variants appearing in sequences affect gene functions, the apparatus (100) comprising:

   a pre-processor (110) that analyzes sequences of samples of genes and extracts data of genetic variants mapped to the genes;
   a toxicity prediction unit (120) that obtains toxicity scores obtained by quantifying genetic dysfunctions affected by the data of genetic variants; and
   a modularization unit (140) that searches for at least one sub-module including a set of genes whose toxicity scores exceed a predetermined critical value from a genetic network.

2. The apparatus (100) of claim 1, wherein the pre-processor (110) comprises:

   a disease group comparison unit (112) that compares genetic variants in a disease group with genetic variants in a normal group and acquires the genetic variants in the disease group from the analyzed gene samples;
   a variant extraction unit (114) that extracts new genetic variants from the acquired disease group variants by referring to known variant database (115); and
   a variant mapping unit (116) that maps the extracted new genetic variants to functional genes.

3. The apparatus (100) of claim 2, wherein the variant mapping unit (116) maps the extracted new genetic variants to the functional genes by extracting only the extracted new genetic variants having amino acids changing when expressed in a protein.

4. The apparatus (100) of claim 1, wherein the toxicity prediction unit (120) comprises a toxicity calculation unit (170) that applies the data of genetic variants to a plurality of toxicity prediction models (175, 176, 177) to obtain the respective toxicity scores, and assigns weights to the respective toxicity scores to obtain weighted toxicity scores.

5. The apparatus (100) of claim 4, wherein the toxicity calculation unit comprises:

   a feature vector generation unit (171) that generates feature vectors including various factors from the data of genetic variants;
   an adapter (172, 173, 174) that sorts factors necessary for the respective prediction models (175, 176, 177) from the generated feature vectors;
   two or more prediction models (175, 176, 177) that receive the sorted factors to detect individual non-synonymous single nucleotide polymorphism (nsSNP) in protein sequences; and
   a weight assignment unit (178) that assigns weights to outputs of the prediction models (175, 176, 177) and sums the weights.

6. The apparatus (100) of claim 5, wherein the weight assignment unit (178) normalizes the outputs of the prediction models (175, 176, 177) to values ranging between 0 and 1, multiplies the normalized outputs by weights, sums the multiplication results, and normalizes the summing result to a value ranging between 0 and 1.

7. The apparatus (100) of claim 5, wherein the feature vector includes at least two of conservation scores of amino acids at positions of genes and proteins mapped to genetic variants in various biological species, biochemical hydrophobicity resulting from amino acid substitution, a change in protein structural features, presence or absence of intron splice junction sites, and five prime untranslated region (5'-UTR) variation position.

8. The apparatus (100) of claim 5, wherein each of the prediction models (175, 176, 177) includes at least one of Sorting Tolerant From Intolerant (SIFT), Polymorphism Phenotyping (PolyPhen), and Map Annotator and Pathway Profiler (MAPP).

9. The apparatus (100) of claim 4, wherein the toxicity prediction unit (120) further comprises:

   a significance calculation unit (180) that calculates significance of a corresponding genetic variant based on the frequency of the data of genetic variants; and
   a score computation unit (190) that combines the weighted toxicity scores and the significance and computes toxicity scores.

10. The apparatus (100) of claim 9, wherein the significance calculation unit (180) calculates the significance based on the probability of detecting genetic variants of the corresponding gene from the disease group variants, and the probability is obtained by maximum likelihood estimation or Bayesian probability estimation.

11. The apparatus (100) of claim 9, wherein the score computation unit (190) obtains a final toxicity score by dividing a sum of toxicity scores of the genetic variants in a single gene by a gene length.

12. The apparatus (100) of claim 1, wherein the modularization unit (140) searches for the sub-modules by repeating an updating process of a genetic network based on whether a merging of a set of current gene nodes with an adjacent gene is significant.

13. The apparatus (100) of claim 12, wherein the modularization unit (140) determines the significance using a probability obtained from a hypergeometic distribution indicating the number of genes whose toxicity scores exceed a predetermined critical value.

14. The apparatus (100) of claim 13, wherein the predetermined critical value is determined based on a predetermined percentile in a toxicity score distribution for entire genes.

15. The apparatus (100) of claim 1, further comprising
   a network merging unit (130) that merges proteins manifested from the genes whose toxicity scores are obtained by the toxicity prediction unit (120) with proteins from the known interaction database (135) to generate an interaction network; or
   a priority determination unit (150) that determines an order of priority in the plurality of sub-modules searched by the modularization unit (140) based on Z-scores; or
   a verification unit (160) that evaluates functional relevance of the sub-modules by comparing the sub-modules arranged by the order of priority with the known pathway database (165).

# FIG. 1

BIOMARKER EXTRACTING APPARATUS(100)

GENE SAMPLE → PRE-PROCESSOR(110)

DATA OF
VARIANTS

TOXICITY PREDICTION
UNIT(120)

TOXICITY
SCORE

NETWORK MERGING
UNIT(130) ⟷ INTERACTION DB(135)

MODULARIZATION
UNIT(140)

PRIORITY DETERMINATION
UNIT(150)

VERIFICATION UNIT(160) ⟷ PATHWAY DB(165)

BIOMARKER

# FIG. 2

# FIG. 3

# FIG. 4

FIG. 5

# FIG. 6

```
                    ┌─────────────┐
                    │    START    │
                    └─────────────┘
                           │
                           ▼
      ┌──────────────────────────────────────┐
      │      SET INITIAL SUB-NETWORK          │ ⟋ S1
      └──────────────────────────────────────┘
                           │
                           ▼
      ┌──────────────────────────────────────┐
      │  SELECT ADJACENT GENE AND GENERATE NEW │ ⟋ S2
      │              NETWORK                   │
      └──────────────────────────────────────┘
                           │
                           ▼
      ┌──────────────────────────────────────┐
      │  EVALUATE SIGNIFICANCE OF NEW NETWORK  │ ⟋ S3
      └──────────────────────────────────────┘
                           │
                           ▼
                        ◇ S4
                   NEW NETWORK            NO
                   ACCEPTED?        ─────────►
                           │
                          YES
                           ▼
      ┌──────────────────────────────────────┐
      │        UPDATE GENETIC NETWORK          │ ⟋ S5
      └──────────────────────────────────────┘
                           │
                           ▼
                        ◇ S6
                   TERMINATION             NO
                   CONDITION        ─────────►
                           │
                          YES
                           ▼
                    ┌─────────────┐
                    │     END     │
                    └─────────────┘
```

# FIG. 7

TOTAL NUMBER OF GENES SEARCHED(N)

NUMBER OF GENES IN
SUB-MODULE SEARCHED
(m)

k

NUMBER OF GENES
ASSOCIATED WITH
PARTICULAR BIOLOGICAL
FUNCTION
(n)

Europäisches Patentamt

European Patent Office

Office européen des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 11 18 6897

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ETHAN CERAMI ET AL: "Automated Network Analysis Identifies Core Pathways in Glioblastoma", PLOS ONE, vol. 5, no. 2, 1 January 2010 (2010-01-01), page E8918, XP55032232, ISSN: 1932-6203, DOI: 10.1371/journal.pone.0008918 * the whole document * | 1-15 | INV. G06F19/18 |
| A | A. TORKAMANI ET AL: "Identification of rare cancer driver mutations by network reconstruction", GENOME RESEARCH, vol. 19, no. 9, 1 September 2009 (2009-09-01), pages 1570-1578, XP55032234, ISSN: 1088-9051, DOI: 10.1101/gr.092833.109 * the whole document * | 1-15 | |
| | -/-- | | |

-----

-----

**TECHNICAL FIELDS SEARCHED (IPC)**

G06F

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 10 July 2012 | Kürten, Ivayla |

EPO FORM 1503 03.82 (P04E07)

Europäisches
Patentamt

European
Patent Office

Office européen
des brevets

**PARTIAL EUROPEAN SEARCH REPORT**

Application Number

EP 11 18 6897

| | **DOCUMENTS CONSIDERED TO BE RELEVANT** | | CLASSIFICATION OF THE APPLICATION (IPC) |
|---|---|---|---|
| Category | Citation of document with indication, where appropriate, of relevant passages | Relevant to claim | |
| A | D. NAM ET AL: "GSA-SNP: a general approach for gene set analysis of polymorphisms", NUCLEIC ACIDS RESEARCH, vol. 38, no. Web Server, 1 July 2010 (2010-07-01), pages W749-W754, XP55032236, ISSN: 0305-1048, DOI: 10.1093/nar/gkq428 * the whole document * | 1-15 | |
| A | M. HOLDEN ET AL: "GSEA-SNP: applying gene set enrichment analysis to SNP data from genome-wide association studies", BIOINFORMATICS, vol. 24, no. 23, 1 December 2008 (2008-12-01), pages 2784-2785, XP55032237, ISSN: 1367-4803, DOI: 10.1093/bioinformatics/btn516 * the whole document * | 1-15 | TECHNICAL FIELDS SEARCHED (IPC) |

EPO FORM 1503 03.82 (P04C10)

Europäisches
Patentamt

European
Patent Office

Office européen
des brevets

**INCOMPLETE SEARCH
SHEET C**

Application Number

EP 11 18 6897

Claim(s) completely searchable:
        -

Claim(s) searched incompletely:
        1-15

Reason for the limitation of the search:

1.1        The claimed subject-matter is unclear (Article 84 EPC). The reasons are as follows:
1.2        Claim 1:
Claim 1 is directed to an apparatus for extracting "causal biomarkers of a specific disease". However, the result of the method is at least one sub-module including a set of genes. It is unclear how said at least one sub-module is related to causal biomarkers of a disease. This lack of correspondence between the aim of the claim and its result renders the scope of protection sought unclear.
It is unclear what is meant by mapping in the expression "genetic variants mapped to the genes".
The function of the "modularization unit" is entirely unclear. Said unit searches for at least one "sub-module". The term "sub-module" is, however, undefined: sub-module of what (of a genetic network)? how is a sub-module defined (e.g. compared to an arbitrary collection of genes)?
Claim 1 states that the at least one sub-module includes a "set of genes whose toxicity scores exceed a predetermined critical value". However, the toxicity prediction unit does not define "toxicity scores" with respect to genes but with respect to genetic variants. Hence, it is unclear what is meant by a toxicity score of a gene.
The genetic network mentioned in claim 1 is not defined either. How are the nodes and the edges of said network defined?
Even with regard to the description (e.g. pages 14 to 17), the subject-matter of claim 1 could not be unambiguously interpreted.
The term "genetic network" seems to be introduced for the first time in paragraph 72 with regard to figure 6. Said paragraph mentions an "initial network" having all genes having significant toxicity scores as single nodes. However, said paragraph does not explain how the edges between the nodes are defined.
Furthermore, the identification of modules is defined with regard to unclear terms such as "current gene", "adjacent gene" and "gene manifestation data". Paragraphs 75 merely states that "sub-modules included in the finally updated genetic network may be determined" without specifying any criteria for the definition of sub-modules. Are "sub-modules" equivalent to the "units composed of the merged nodes" (paragraph 73)?
Finally, the only sentence in the entire description that links biomarkers to sub-modules is on page 17, lines 8 - 10. According to said sentence "the sub-modules ... may function as biomarkers indicating correlation to manifestation of a particular gene". Said sentence, however, still does not give an unambiguous interpretation about the relationship between biomarkers and selected sub-modules. May all the genes in a sub-modules function as biomarkers?
1.3        With regard to claim 2, it is unclear whether and how the acquisition of the genetic variants in the disease group depends on the comparison of genetic variants in a disease and a normal group. Said

Europäisches
Patentamt

European
Patent Office

Office européen
des brevets

**INCOMPLETE SEARCH
SHEET C**

Application Number

EP 11 18 6897

claim further aims at the extraction of "new genetic variants ... by referring to known variant database". It is unclear what "new genetic variants" means (new with respect to what?)?

2.1      Given the extent of the clarity objections, no meaningful search of the claimed subject-matter could be performed.

2.2      In the letter of 02.05.2012, the applicant has responded to the invitation pursuant to Rule 63(1) EPC. The applicant has requested to perform the search with respect to an apparatus comprising a number of modules that function as specified in said letter. The indication of the applicant cannot be fully taken into account because it is based on passages with unclear or vague wordings.

1.1      Taking into account the objections in par. 2 above and the observations in par. 3.2 above, the subject-matter of the application has been interpreted broadly. The search for prior art has been conducted with respect to a method and an apparatus for identifying new genetic variants specific to a given disease. From the identified variants only those are selected that have some functional impact (i.e. non-synonymous SNPs). Genetic dysfunctions are quantified ("toxicity scores" derived) based on the selected variants. The genes are analyzed in the context of a genetic network to identify sub-networks that are enriched with genes having high toxicity scores.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020100105504

**Non-patent literature cited in the description**

- *Automated Network Analysis Identifies Core Pathways in Clioblastoma,* February 2010, vol. 5 (2), e8918, www.plosone.org